# EUROPEAN PATENT APPLICATION

(11) **EP 4 674 422 A1**
(43) Date of publication of application: **07.01.2026**
(21) Application number: 24186349.7
(22) Date of filing: 03.07.2024
(51) Int. Cl.: A61K 36/185, A61K 36/31, A61K 38/47, A61K 47/00, A61P 31/00, A61P 31/04, A61P 31/10, A61P 31/12, A61K 31/095

(54) **MUSTARD OIL GLYCOSIDE- AND MYROSINASE-COMPRISING PLANT EXTRACTS**

(71) Applicant: Max Zeller Söhne AG, 8590 Romanshorn (CH)
(72) Inventor: BOONEN, Georg, 8590 Romanshorn (CH); Kreuter, Matthias-Heinrich, 8880 Walenstadt (CH); Kreuter, Emanuel Nikolaus, 9470 BUCHS (CH)
(74) Representative: Kasche & Partner

(57) **Abstract**

The present invention is directed to a dried plant extract mix comprising (i) a dried extract component comprising mustard oil glycosides (glucosinolates), and (ii) a dried extract component of active myrosinase enzymes, or isolated active myrosinase enzymes, wherein the extract component (i) comprising mustard oil glycosides does not comprise active myrosinase enzymes. The invention further relates to methods for preparing the dried extracts, medical compositions comprising the dried extracts and medical uses thereof.

## Description

The present invention is directed to a dried plant extract mix comprising (i) a dried extract component comprising mustard oil glycosides (glucosinolates), and (ii) a dried extract component of active myrosinase enzymes, or isolated active myrosinase enzymes, wherein the extract component (i) comprising mustard oil glycosides does not comprise active myrosinase enzymes. The invention further relates to methods for preparing the dried extracts, medical compositions comprising the dried extracts and medical uses thereof.

### STATE OF THE ART

Mustard oil glycosides are an inherent defense system in plants, in particular in plants of the family Brassicaceae (previously Crucifera), an economically important family of flowering plants commonly known as the mustards, the crucifers, or the cabbage family. Further mustard oil glycoside-producing plant families are Capparaceae, Caricaceae, Putranjivaceae and Tropaeolaceae.

For example, Brassicaceae encompasses but is not limited to horseradish root (Armoracia rusticana), and Tropaeolaceae includes Nasturtium herb (Nasturtium Tropaeolum majus Herba). The mustard oil glycosides, so called glucosinolates release isothiocyanates (aglycans) upon enzymatic removal of the sugar component. The isothiocyanates of the Brassicaceae and Tropaeolaceae families are known to have a broad spectrum of antimicrobial activity against bacteria, viruses and fungi.

In intact plant tissue the glucosinolates and myrosinase enzymes are located in separate plant cells and react upon destruction of this compartimentation (Underhill EW 1980 Glucosinolates. In: Bell EA, Charlwood BV (Hrsg.) Secondary Plant Products, Springer, Berlin Heidelberg New York, p. 493-511). The release of the isothiocyanates can be achieved either by fermentation of fresh, finely comminuted plant or by enzymatic reaction of dried and pulverized drug in aqueous solution at room temperature (RT). Heat inactivates the myrosinases and so do high alcohol concentrations such as 70% v/v methanol (Kjaer A, Madsen JO, Maeda Y (1978) Phytochemistry 17: 1285-1287).

Presently, about 130 different chemically different glucosinolates are known (Angerbirk & Olsen, 2012). All presently known plant myrosinases belong to the glucosylhydrolyase-family 1, which encompasses procaryotic as well as eucaryotic enzymes such as, example given, O-β-glucosidases, 6-phospho-β-glucosidases, 6-phospho-β-galactosidases and lactases. The classical myrosinase enzymes comprise conserved aglycon-binding amino acid residues as well as a conserved glutamate residue (acidic /basic glutamate) in the active center. Myrosinases are strongly glycosylated, mostly dimeric proteins and utilize ascorbate as co-factor (Andreas Loebers, PhD thesis at Friedrich-Alex-ander-Universität Erlangen-Nürnberg, "Molekularbiologische und biochemische Analysen zum Glucosinolat-Myrosinase-System und der Agrarbiologie der Meerrettichpflanze (Armoracia rusticana Gaertn., Mey. & Scherb.", 24.07.2014, p. 1 to 6)).

A therapeutic effect in medical preparations against bacterial infections in a human or animal requires concentrations of isothiocyanates that inhibit growth (bacteriostatic) or survival of bacteria (bacteriocidal). The isothiocyanates are volatile, sensible to oxygen and light, unstable, and age upon storage that leads to a loss of efficacy (Dannenberg H, Stickl H, Wenzel F (1956) Hoppe-Seylers Z. Physiol Chem 303; 248-256).

Medical plant preparations typically contain finely ground powder of dried plants of Brassicaceae in tablet form. One such example is Angocin^{®} which contains 80 mg Armoracia rusticana and 200 mg Nasturtium per tablet and which is recommended for therapy of uncomplicated urinary infections (see Interdisziplinäre S3 Leitlinie, »Epidemiologie, Diagnostik, Therapie, Prävention und Management un-komplizierter, bakterieller, ambulant erworbener Harnwegsinfektionen bei erwachsenen Patienten», AWMF-Register-Nr.043/044). For Angocin^{®} the daily dosage is 3 to 5 times 4 to 5 tablets. The intake of such a large amount of tablets for treating even uncomplicated infections limits the application significantly due to compliance problems and rules out the treatment of severe infections such as, for example, problematic multi-resistant germs.

There are also liquid preparations such as tinctures and mother tincture that are mainly prepared from fresh plant parts with aqueous ethanol preparations (43-84 % v/v ethanol). An example of these liquid preparations is Sagrusan^{®} (tincture of Nasturtium) or the mother tincture made from Armoracia according to HAB 34 (Urtinktur und flüssige Verdünnungen nach HAB 1, Vorschrift 3 a. Armoracia horn. HAB 34). These products have significantly lower drug concentrations than simple powder drugs. Due to their preparation method they do not comprise intact myrosinase enzymes (low dissolution and denaturation in the extraction medium). Under these storage conditions liquid plant preparations undergo accelerated degradation. For this reason, liquid medical preparations of Brassicaceae glucosinolates are essentially useless for the systemic treatment of bacterial infections.

### OBJECTIVE OF THE INVENTION

It is an aim of the present invention to provide a dry extract from glucosinolate and myrosinase-comprising plants which contains both the precursors of the isothiocyanates, namely the glucosinolates, in a high concentration compared to drug powder content, and active myrosinase enzymes. Optionally, it should contain both aromatic and aliphatic glucosinolates. Furthermore, both the glucosinolates, which are storage-stable as such, and the myrosinases required for their cleavage must be present in a stable and active form and only release the isothiocyanates completely or almost completely on contact with suitable aqueous media.

It is a further objective to provide an efficient and improved medicament against bacterial, viral and fungal infections in humans and animals, with good compliance and low daily dosages in high concentrations.

The objectives of the present invention are solved by a dried plant extract mix comprising
(i) a dried extract component comprising mustard oil glycosides (glucosinolates), and
(ii) a dried extract component comprising active myrosinase enzymes, or isolated active myrosinase enzymes;
wherein the extract component (i) comprising mustard oil glycosides (glucosinolates) does not comprise active myrosinase enzymes.

The inventive plant extract mix of the present invention is characterized by a glucosinolate-comprising dried extract component (i) and a dried extract component (ii) comprising active myrosinase enzymes in the absence of amounts water so that the extract mix is inactive until (further) water is added. When both extract components are prepared separately and dried before mixing, the myrosinase enzymes remain inactive, storage stable and ready for use by water addition, and the glucosinolates remain storage-stable too.

Optionally, the glucosinolate extract component (i) will also comprise the plant's inherent ascorbate / ascorbic acid content, which is co-extracted and will function as co-factor for the enzymatic isothiocyanate release. Optionally, the dried myrosinase extract component may comprise some remaining glucosinolates. However, as the active myrosinase extract component is typically depleted of the co-factor ascorbate, these remaining glucosinolates typically remain untransformed until the co-extracted ascorbate from the glucosinolate extract (i) and water are added. Alternatively, if the inventive dried plant extract mix is prepared without ascorbate/ascorbic acid content or too little ascorbate/ascorbic acid content, myrosinase activation will require the addition of water and ascorbate and/or ascorbic.

Because the preparation of the dried extract component (i) requires the removal of myrosinase enzymes, it is storage-stable, even in the presence of the myrosinase co-factor ascorbate. Because the dried extract component (ii) either lacks ascorbic acid/ascorbate and/or glucosinolates, it is storage stable too. Alternatively, the dried myrosinase extract component (ii) may be isolated active myrosinase enzymes, for example, recombinantly produced myrosinases or myrosinases from a plant extract but further isolated, e.g. by cut-off filtration of the enzymes.

The term "dried plant extract mix", as used herein, refers to a water content in the plant extract mix of the invention, at which the myrosinase enzymes remain inactive in the extract mix until water addition. Optionally, the water content in the dried plant extract mix of the present invention is less than or equal 15, optionally less than or equal 10, optionally less than or equal 5 or 1 % m/m water.

The term "dried extract components", as used herein, is meant to indicate a water content in the extract components (i) and (ii), at which the myrosinase enzymes remain inactive in the extract components until further water addition. In the plant extract mix of the present invention, the water content of the dried extract components (i) and (ii) is less than or equal 15, optionally less than or equal 10, optionally less than or equal 5 or 1 % m/m water.

It has been found that one or both of the extract components (i) and (ii) may comprise up to 30, up to 40, or even up to 50 % by weight water without significantly initiating myrosinase activity for some time. Hence, one or both of the dried extract components (i) and (ii) may comprise up to 30, 40 or even 50% m/m water before or when being combined and then further dried to prepare the dried plant extract mix of the invention. The water content in the plant extract components (i) and (ii) at which myrosinases remain inactive for some time will depend on the extractant, its organic solvent component(s), the water content, the extract composition, the particular plant species, extraction particulars and more factors.

Optionally, the plant extract mix of the present invention is one, wherein
(a) the water content in the dried plant extract mix of the present invention is less than or equal 15, optionally less than or equal 10, optionally less than or equal 5 or 1 % m/m water; and/or
(b) the water content in the dried plant extract components (i) and (ii) before and/or during mixing is less than or equal 50, optionally less than or equal 40, optionally less than or equal 30 % m/m water.

The term "active myrosinase enzyme", as used herein, is as understood in the art, i.e. that the myrosinase enzyme can release isothiocyanates from glucosinolates in aqueous medium in the presence of required co-factors, e.g. ascorbate/ascorbic acid.

The term "isolated active myrosinase enzyme", as used herein, is meant to encompass those active myrosinase enzymes that are free of most or all further extract components, due to further isolation procedures or due to recombinant production.

For example, the dried extract components (i) and (ii) of the inventive dried plant extract mix can be prepared from the same or different plant materials.

Optionally, the dried plant extract mix according to the present invention is one, wherein the dried plant extracts (i) and (ii) were prepared from different plant materials. When preparing the components of the dried plant mix from different plant materials, the yield of glucosinolates and the myrosinases can be optimized, for example, because glucosinolates can be extracted under conditions that inactivate the myrosinases.

Alternatively, the dried plant extract mix of the present invention may be prepared from the same plant materials by sequential extraction, optionally a first extraction of the mustard oil glycol-sides, and a second extraction of active myrosinase enzymes from the retentate residue of the first extraction.

In principle, the dried plant extract mix can be prepared from any plant material comprising glucosinolates and/or myrosinase enzymes. For example, the dried plant extract mix of the invention may be prepared from mustard oil (glucosinolate)- and myrosinase-comprising plants of the families Brassicaceae, Capparaceae, Caricaceae, Putranjivaceae and Tropaeolaceae.

Optionally, the dried plant extract mix comprises extract material from plants selected from the group consisting of
(i) Brassicaceae species, e.g.
   white cabbage, (Brassica oleracea var. capitata f. alba); brussels sprouts (Brassica oleracea var. gemmifera); broccoli (Brassica oleracea var. italica); cauliflower (Brassica oleracea var. botrytis); radish (Raphanus sativus); horseradish (Armoracia rusticana); garden cress (Lepidium sativum); mustard (Sinapis alba, Sinapis nigra); rapeseed (Brassica napus); rucola (Arugula), Eruca sativa; Brassica oleracea var. gongylodes; savoy cabbage (Brassica oleracea var. sabauda), kale (Brassica oleracea var. sabellica); bok choy (Brassica rapa subsp. chinensis); wasabi (Eutrema japonicum); Daikon radish (raphanus sativus var. longipinnatus);
(ii) Capparaceae species, e.g. Capparis spinosa et var.;
(iii) Tropaeolaceae species, e.g. Tropaeolum majus et var. ;
(iv) Caricaceae species, e.g. Carica papaya et var.; and
(v) Putranjivaceae, e.g.Drypetes separia et var.

For example, the dried plant extract mix of the invention may optionally have
- a total content of mustard oil glycosides (glucosinolates) of 1 to 900, optionally 30 to 600, optionally 80 to 400 mg mustard oil glycosides per g dried extract component (i), and/or
- a total content of myrosinase enzymes of 0.01 units to 100.000 units, optionally 1 unit to 1000 unit, optionally 50 to 250 units per g dried extract component (ii).

For medical use it may be beneficial if the drug extract ratio (DER) of
- the extract component (i) of mustard oil glycosides (glucosinolates) is, for example, 2:1 to 50:1, optionally 5:1 to 16:1, optionally 4:1 to 10:1; and
- the extract component (ii) of myrosinase enzymes, or isolated myrosinase enzymes is, for example, 2:1 to 200:1, optionally 50:1 to 200:1, optionally 10:1 to 30:1.

When preparing the extract components (i) and (ii) from the same plant material one has to consider that the myrosinase enzymes are likely to cleave the glucosinolates quickly into isothiocyanates in aqueous medium when the water content in the extract rises above about 30, 40 or 50 % m/m once the cell walls of the plant cells no longer separate these components. On the other hand, organic solvents tend to denature the enzymes. For this reason, it is important to practice a sequential and separate extraction of the two extract components from the same plant material, for example obtaining the glucosinolate extract component (i) first and the myrosinase extract component (ii) secondly.

In another aspect, the present invention pertains to methods for preparing the dried plant extract mix of the present invention.

For example, the dried plant extract mix of the present invention can be prepared from the same plant material by a method, comprising the following steps:
A Extraction of mustard oil glycosides (glucosinolate extract)
   (a) extraction of plant material in 5 to 85, 10 to 67, 20 to 66, or 40 to 68 m/m aqueous organic solution, optionally alcohol, aldehyde or ketone-comprising aqueous organic solution, optionally comprising MeOH, EtOH, nPrOH, iPrOH, acetaldehyde, propanal, dimethylketone, ethylmethyl ketone, diethylketone, optionally EtOH, at 0 to 75 or 20 to 40°C, optionally at a pH of 3 to 10, or 6 to 9;
   (b) separating the plant material from the liquid extract and evaporation of the liquid extract at 20 to 90 or 30 to 60°C, optionally at reduced pressure, optionally diluting with 0 to 35% m/m aqueous organic solution, optionally EtOH, filtration and evaporation of the filtrate;
   (c) drying the filtrate, thereby producing extract component (i) (glucosinolates);
B Extraction of myrosinase enzymes (myrosinase extract)
   (a) suspending the residue plant material from Extraction A in 0 to 68, 10 to 30, 15 to 25 or about 20 % m/m organic solution, optionally alcohol, aldehyde or ketone-comprising aqueous organic solution, optionally comprising MeOH, EtOH, nPrOH, iPrOH, acetaldehyde, propanal, dimethylketone, ethylmethyl ketone, diethylketone, optionally EtOH, optionally at a pH of 3 to 10, or at a pH of 7 to 9;
   (b) optionally micronization of the suspended plant material;
   (c) extraction of the suspended plant material at 0 to 50 or 10 to 42 C° for at least 5, 10, 20, or at least 30 min;
   (d) filtration;
   (e) optionally subjecting the filtrate of (d) to a 20 to 300, 40 to 100, 45 to 55, or an about 50 kD cut off membrane filtration; (leaving the enzyme in the retentate, smaller molecules in the permeate. Myrosinases typically have a molecular weight of at least 120-580 kD depending on dimerization, hydration status, etc.)
   (f) optionally adding a protein stabilizing agent to the filtrate of (d) or the retentate residue of (e), optionally maltodextrin;
   (g) evaporation and drying the filtrate of (d), the retentate residue of (e) or (f), thereby producing extract (ii) (myrosinases);
C Combination of the dry glucosinolate and the dry myrosinase extract components (i) and (ii), and optionally adding a dispersing agent, optionally a highly disperse silicon dioxide, optionally adding ascorbic acid or ascorbate, optionally further drying the extract mix.

It is again noted that the dried plant extract mix of the invention should optionally have a water content of equal or less than 15, 10, 5 or 1 % m/m water. It is also noted that the dried glucosinolate and dry myrosinase extract components (i) and (ii) should optionally have a water content of equal or less than 50, 40, or 10% m/m water before and/or during mixing.

For optimizing yields and other reasons, the dried extract mix of the present invention may be prepared from different plant materials, optionally from the same or different plant species. For example, the dried plant extract mix of the present invention may be prepared from different plant materials by a method comprising the following steps:
A Extraction of mustard oil glycosides (glucosinolate extract)
   (a) extraction of plant material in 0 to 95, 10 to 80, 30 to 75, or 40 to 70 m/m aqueous organic solution, optionally alcohol, aldehyde or ketone-comprising aqueous organic solution, optionally comprising MeOH, EtOH, nPrOH, iPrOH, acetaldehyde, propanal, dimethylketone, ethylmethyl ketone, diethylketone, optionally EtOH, at 5 to 80 or 20 to 60°C, optionally at a pH of 1 to 12, or 5 to 7;
   (b) separating the plant material from the liquid extract and evaporation of the liquid extract at 20 to 90 or 30 to 60°C, optionally at reduced pressure, optionally diluting with 0 to 35% m/m aqueous organic solution, optionally EtOH, filtration and evaporation of the filtrate;
   (c) drying the filtrate(s), thereby producing extract component (i) (glucosinolates);
B Extraction of myrosinase enzymes
   (a) suspending plant material not used in Extraction A in 0 to 68, 10 to 30, 15 to 25 or about 20 % m/m aqueous organic solution, optionally alcohol, aldehyde or ketone-comprising aqueous organic solution, optionally comprising MeOH, EtOH, PrOH, iPrOH, acetaldehyde, propanal, dimethylketone, ethylmethyl ketone, diethylketone, optionally EtOH, optionally at a pH of 3 to 10, or at a pH of 5 to 9;
   (b) optionally adding 1 to 20% m/m of a lipophilic phase relative to the aqueous phase of (a), optionally hydrocarbons, oils, waxes, optionally hexane, heptane, octane, plant oil, plant wax, bee wax; (the term "lipophilic phase relative to the aqueous phase" used in step (a) is meant to indicate that the lipophilic phase is more lipophilic than the aqueous phase. The lipophilic phase serves the extraction of released isothiocyanates into the lipophilic phase);
   (c) optionally micronization of the suspended plant material;
   (d) extraction of the suspended plant material at 0 to 50 or 10 to 42 C° for at least 5, 10, 20 or at least 30 min;
   (e) filtration, optionally microfiltration through a 0.01-10 µm cut off filter for separating the lipophilic phase of (b),
   (f) optionally subjecting the filtrate of (e) to a 10 to 300, 40 to 100, 45 to 55, or an about 50 kD cut off membrane filtration and collecting the retentate comprising the myrosinases;
   (g) optionally adding a protein stabilizing agent, optionally maltodextrin, to the filtrate of (e) or the retentate residue of (f);
   (h) evaporation and drying the filtrate of (e), the retentate residue of (f) or (g), thereby producing extract component (ii) (myrosinases);
C Combination of the dry glucosinolate and the dry myrosinase extract components (i) and (ii), and optionally adding a dispersing agent, optionally a highly disperse silicon dioxide, optionally adding ascorbic acid or ascorbate, and optionally further drying the plant extract mix.

In a further alternative, the dried plant extract mix of the present invention may comprise isolated myrosinase enzymes, i.e. not forming part of an extract. In an exemplary embodiment, the extract mix of the invention may be prepared from (i) an extract comprising mustard oil glycosides (glucosinolates), and
(ii) isolated myrosinase enzymes, preparable from plant material, animal or microbial material (fungal, bacterial) or prepared recombinantly;
by combination of the extract (i) and the isolated myrosinase enzymes (ii), wherein extract (i) does not comprise active myrosinase enzymes.

The term "isolated myrosinase enzymes", as used herein, is meant to encompass those myrosinase compositions which are essentially free of plant extract components, e.g. have less than 10, 8, 5, 2 or less than 1 % w/w plant-derived non-myrosinase materials in the dried myrosinase composition.

In the following, an optional method for preparing a dried plant extract mix of the present invention from the same plant material of Nasturtium tropaeolum (Tropaeolum majus) is described, comprising the following steps
A Extraction of mustard oil glycosides (glucosinolate extract)
   (a) extraction of Nasturtium plant material in 60 to 68% m/m EtOH, optionally at a pH of 2 to 4;
   (b) filtration and evaporation of the filtrate of (a) to at least 20% of the extraction volume;
   (c) re-dilution of filtrate (b) with 0 to 20% m/m EtOH, filtration, optionally filtration through a 1 to 50 kD cut off membrane;
   d) evaporation and drying the filtrate of (c), thereby producing extract component (i) (glucosinolates);
B Extraction of myrosinase enzymes (myrosinase extract)
   (a) resuspending the sediment from A in 0 to 68 %m/m EtOH,
   (b) optionally adding 1 to 20% m/m of a lipophilic phase relative to the aqueous phase;
   (c) optionally micronization of the suspended plant material;
   (d) extraction of the suspended plant material at 0 to 42C° for at least 5, 10, 20 or at least 30 min;
   (e) optionally subjecting the filtrate of (d) to 20 to 300, 40 to 100, 45 to 55, or an about 50 kD cut off membrane filtration and collecting the retentate comprising the myrosinases;
   (f) optionally adding a protein stabilizing agent, optionally maltodextrin, to the filtrate of (d) or the retentate residue of (e);
   (g) evaporation and drying the filtrate of (d), the retentate residue of (e) or (f), thereby producing extract component (ii) (myrosinases);
C Combination of the dry glucosinolate and the dry myrosinase extract components (i) and (ii), and optionally adding a dispersing agent, optionally a highly disperse silicon dioxide, optionally adding ascorbic acid or ascorbate, and optionally further drying the plant extract mix.

In the following, an optional method for preparing a dried plant extract mix of the present invention from the same plant material of Armoracia is described, comprising the following steps
A Extraction of mustard oil glycosides (glucosinolate extract)
   (a) extraction of Armoracia plant material in 45 to 60% m/m EtOH, optionally at a pH of 2 to 10;
   (b) filtration and evaporation of the filtrate of (a) to at least 20% of the extraction volume;
   (c) re-dilution of filtrate (b) with 0 to 20% m/m EtOH, filtration, optionally filtration through a 1 to 50 kD cut off membrane;
   d) evaporation and drying the filtrate of (c), thereby producing extract component (i) (glucosinolates);
B Extraction of myrosinase enzymes (myrosinase extract)
   (a) Resuspending the sediment from A in 0 to 35%m/m EtOH, optionally at a pH of 5 to 9;
   (b) optionally adding 1 to 20% m/m of a lipophilic phase relative to the aqueous phase;
   (c) optionally micronization of the suspended plant material;
   (d) extraction of the suspended plant material at 0-42C° for at least 5, 10, 20 or at least 60 min and filtration;
   (e) optionally subjecting the filtrate of (d) to 20 to 300, 40 to 100, 45 to 55, or an about 50 kD cut off membrane filtration and collecting the retentate comprising the myrosinases;
   (f) optionally adding a protein stabilizing agent, optionally maltodextrin, to the filtrate of (e) or the retentate residue of (f);
   (g) evaporation and drying the filtrate of (d), the retentate residue of (e) or (f), thereby producing extract (ii) (myrosinases);
C Combination of the dry glucosinolate and the dry myrosinase extract components (i) and (ii), and optionally adding a dispersing agent, optionally a highly disperse silicon dioxide, optionally adding ascorbic acid or ascorbate, and , and optionally further drying the plant extract mix.

In a further aspect, the present invention relates to the dried plant extract mix of the invention, preparable by a method of the invention as exemplified above and in the examples below.

Due to the medical utility of the plant extract mix of the present invention, a third aspect relates to medical, i.e. pharmaceutical compositions comprising the dried plant extract mix of the invention, and optionally at least one physiologically acceptable excipient, optionally selected from the group consisting of protein stabilizing agents, optionally selected from the group consisting of maltodextrin, trehalose, cyclodextrin, dextrose, dispersing agents, optionally selected from the group consisting of silicon dioxide, highly disperse silicon dioxide, calciumphosphate, calciumcarbonate, magnesiumcarbonate, citric acid, citrate, ascorbic acid, and ascorbate.

The dried plant extract mix of the invention and the pharmaceutical composition of the invention are intended for use as a medicament, optionally for use in the treatment or prevention of a bacterial, viral or mycotic infection. The isothiocyanates resulting from the dried plant extract mix tend to be generally toxic against bacteria, viruses and fungus, however, less toxic in mammals and humans.

Optionally, the dried plant extract mix or pharmaceutical composition for use according to the invention is one, wherein the bacterial, viral or mycotic infections are selected from
a. bacterial infections, selected from the group consisting of
   MRSA (multiple drug-resistant staphylococcus aureus), optionally Staphylococcus aureus;
   MDR (multiple drug-resistant) bacteria, optionally Staphylococcus aureus, Clostridium difficile, Streptococcus pneumoniae;
   Mycobacteria sp, optionally tuberculosis, abscessus, leprae;
   ESBL (extended spectrum beta lactamase) bacteria, optionally Enterobacter sp., Salmonella sp., Proteus sp., Serrama marcescens, Shigella dysenteria, Burkholderia cepacia; and
   CPE (carbapenemase producing enterobacteriae) resistant bacteria, optionally Escherichia coli, Klebsiella pneumoniae, Acinetobacter baumannii, Pseudomonas aeroginosa;
   viral infections, selected from the group consisting of influenza virus, corona virus, rotavirus, enzephalitis virus, Norovirus; and
   mycotic infections, selected from the group consisting of fungis, optionally the genus aspergillus, optionally A. niger, the genus fusarium, optionally fusarium solani, fusarium oxysporum, the genus candida, optionally Candida albicans.

And in another aspect, the present invention relates to a to a method of treatment of bacterial, viral or mycotic infections in a mammal or human, comprising the step of administering a dried plant extract mix or a pharmaceutical composition of the present invention to a mammal, optionally a human in need thereof, in an effective amount.

The term "treatment", as used herein, relates to the prophylactic and/or therapeutic treatment of a disease or medical condition. For example, the extract or composition for use according to the present invention can be administered before or after a bacterial, viral or mycotic infection in order to inhibit and/or alleviate pathogen-induced physiological consequences, in particular pathogen-induced inflammatory reactions.

An extract or a pharmaceutical composition, i.e. a medicament for use according to the invention, typically comprises a dried plant extract mix of the invention in a pharmaceutically effective amount suitable for administration, optionally formulated together with conventional pharmaceutically acceptable additives, carriers, adjuvants and excipients and/or further pharmaceutically active agents. Controlled release dosage forms with or without immediate release portions are also envisaged. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. One skilled in the field of preparing formulations can readily select the proper form and mode of administration for an extract mix for use in the present invention depending upon the particular characteristics of the extract product selected, the bacterial, viral or mycotic disease or infection-related condition to be treated, the stage of the disease or condition, the specific patient and other relevant circumstances (see. e.g. Remington's Pharmaceutical Sciences, Mack Publishing Co. (1990)).

As the person skilled in the art will appreciate, lower or higher doses may be required depending on particular factors. For instance, specific doses and treatment regimens will depend on factors such as the patient's general health profile, the severity and course of the patient's disorder or disposition thereto, and the judgment of the treating physician which includes interaction potentials with other needed medication.

In a preferred embodiment the pharmaceutical composition for use in the present invention comprises 10 to 600 mg plant extract mix, preferably 50 to 500 mg, more preferably 200 to 400 mg, depending of course on the intensity of treatment required.

Although one dose per day may be sufficient, up to 5 or more doses per day may be applied. e.g. orally or topically. Optionally, up to 3 doses per day may be administered. As the person skilled in the art will appreciate, lower or higher doses may be required depending on particular factors.

The extracts for use in the present invention can be administered the same way as other chemical drugs or plant extracts and pharmaceutical compositions thereof.

In a preferred embodiment, the extract or pharmaceutical composition for use according to the invention is administered orally or topically.

It is preferred that the extract or pharmaceutical composition for use according to the invention is a dosage form selected from the group consisting of a spray, a foam, a powder, a tablet, a capsule, a soft gelatin capsule, a tea, a syrup, a granule, a chewable tablet, a salve, a cream, a gel, a lozenge, a liposome composition, preferably a time-release or sustained-release dosage form of the above.

While all types of application of the medicament are believed to be suitable for use according to the invention, oral and topical administrations are preferred for many purposes, and the extract can be used as such or in dilution with solid and/or liquid additives and/or contain additives, carriers, adjuvants, excipients and/or other pharmacologically active constituents.

In a more preferred embodiment, the extract or pharmaceutical composition for use according to the invention is a dosage form selected from the group consisting of a tablet, a capsule, a powder, a granule, a tea, a syrup, an aerosol, a spray, and a time-release or sustained-release dosage form of the above.

Furthermore, the dried plant extract mix of the present invention can be or form part of a food supplement, that has bacteriocidal, antiviral and/or antifungal properties.

Last but not least, the present invention relates to an aqueous or dry plant extract comprising
(i) mustard oil glycosides (glucosinolates), and
(ii) active myrosinase enzymes, or isolated active myrosinase enzymes;
wherein the aqueous or dry plant extract does not comprise ascorbic acid and/or ascorbate. Due to the absence of the enzyme cofactor ascorbic acid/ascorbate in the extract, the myrosinase enzymes cannot cleave the glucosinolates until the co-factor ascorbic acid and/or ascorbate are added. For activation, the dry plant extract requires water and the co-factor, and the aqueous plant extract requires the co-factor. Thus, both the dry and the aqueous options are storage-stable until use. For example, for medical use a liquid aqueous plant extract without co-factor may be co-administered with an ascorbic acid/ascorbate dosage form, for example, in throat sprays, gargle solutions, ear drops, topical antiseptics, sanitizer solutions, bath additives for foot baths, moist compresses, etc.

In the following the invention will be illustrated in more detail by practical examples, none of which are to be interpreted as limiting the scope of the invention beyond the claims as appended.

### Examples

### Dry extracts from nasturtium tropaeolum

### Examples a) to c) - Preparation of glucosinolate-comprising dry extracts from nasturtium tropaeolum (Tropaeolum majus)

Finely cut nasturtium herbs (drug) with a glucosinolate content of 5.75mg/g were extracted with
a) 65% m/m ethanol (50 g herbs),
b) 70% m/m ethanol (100 g herbs), or
c) 75% m/m ethanol (50 g herbs)
in an herb : extraction media ratio of 1:10 at room temperature (RT) for 3 hours with mechanical stirring (300 rpm). The filtrate was filtered via sheet filter.

The filtered liquid extracts were concentrated under vacuum in a rotary evaporator to 30% dry substance content at 50°C and 200 - 30 mbar. The fourfold amount of 20% m/m ethanol was added to the concentrated liquid extracts and stirred for 30 min at 300 rpm and RT. The filtrate was filtered via a sheet filter and dried in a rotary evaporator without any auxiliary additives at 50°C and 200 -1 mbar.

The dry extracts were transferred to a mortar, 0.1% Aerosil^{®} 200 (highly disperse silicon dioxide, Aerosil^{®} 200, Evonik, Germany) were added, the mixture was finely powdered, filled in brown glass bottles and stored tightly closed in an exsiccator.

**Table 1 - Yields of glucosinolate-comprising dry extracts**

| Example | Extract solid yield | Glucosinolate content mg/g |
|---|---|---|
| a) | 8.8 g (DER*:6:1) | 9.2 |
| b) | 14 g (DER: 7:1) | 14.1 |
| c) | 8.2 g (DER: 6:1) | 16.1 |

| | | |
|---|---|---|
| *DER - drug extract ratio | | |

For examples a) and b) the extracted sediments were separated and further extracted for myrosinase enzymes according to Examples d) and e) below.

### Examples d) and e) - Preparation of myrosinase-comprising dry extracts from nasturtium tropaeolum

20% m/m ethanol was added to the above-mentioned pre-extracted herbs (solids) of examples a) and b) in a ratio of 1/10 m/m (10 x EtOH) and these were transferred into separate agitator vessels.

Extraction was implemented at RT under stirring. Subsequently, the pH of the extraction (5.5) was adjusted stepwise with NaOH to 8.5 and extracted for further 60 min. Then the herb solids were size-reduced for 2 min with a Polytron^{®} (PT 1200 E, Kinematica, Switzerlandand) at 5.000 RPM. The resulting solids were again extracted for 60 min and then finely filtered. Each filtrate is verified for its content of extracted material, 50% m/m Maltodextrin relative to the dry substance content is added and dried at 39°C under vacuum (200 to 1 mbar). The resulting dry extracts were transferred to a mortar, 0.1% Aerosil^{®} was added, the mixture was finely powdered, filled in brown glass bottles, and stored tightly sealed in an exsiccator.

### Examples d) and e) - Qualitative test for enzymatic activity of dry extracts d) und e)

400 mg enzyme extract und 600 mg glucosinolate extract of Example c) were dissolved in 30 g drinking water comprising 1 mM ascorbic acid (Ph. Eur.), filled in closed vessel and stirred for 30 min at RT. Then an olfactory test (smelling) was done and sensorically a clearly recognizable release of characteristically smelling isothiocyanates was determined in the myrosinase-comprising extract of Example d). There was no characteristic smell for isothiocyanates in the extract prepared according to Example e). Without wishing to be bound by theory it is assumed that the high ethanol content in the first extraction of Example b) denatured the enzymes. Dissolving only the single extracts d), e) and c) in drinking water did not result in any clearly recognizable smell characteristic for released isothyocyanates.

### Dry extracts from Armoracia rusticana

### Examples a) and b) - Preparation of glucosinolate- and myrosinase-comprising dry extracts from Armoracia rusticana

Extract a): In a first step, 50 g dried and pulverized horse radish root were extracted for a partial extract with 50% m/m Ethanol in a ratio of 1:10 (10 x EtOH), at RT, under stirring at 300rpm for 2h. The filtrate is filtered with a sheet filter and the filtrate is dried at 50°C and 200 to 1 mbar without any auxiliary excipients in a rotary evaporator, resulting in partial extract a).

Extract b): In a second step 20% m/m ethanol in a ratio of 1:10 is added to the remaining filter cake of the powdered horse radish root and extracted at RT under stirring at 300 rpm for 30 min. Then the herb solids were size-reduced for 2 min with a Polytron^{®} (PT 1200 E, Kinematica, Switzerland) at 5.000 RPM and stirred at RT for 30 min at 300 rpm. The solids are filtered via a sheet filter and the filtrate was dried in a rotary evaporator with 50% maltodextrine (relative to the dry extract weight) at 35-40°C, and 200 to 1mbar), resulting in a partial extract b).

Partial extract a) resulted in an extraction yield of 8.5 g (DER:6:1) and partial extract b) in an extraction yield of 2.5 g (DER: 20:1).

Samples of partial extracts a) and b) were mixed homogeneously with addition of 0.5% m/m Aerosil^{®} 200 (highly disperse silicon dioxide, Aerosil^{®} 200, Evonik, Germany) and stored tightly sealed in an exsiccator.

### Qualitative test for enzymatic activity of dry partial extracts a) and b)

1000 mg of the combined partial dry extracts were dissolved in 30 g drinking water comprising 2 mM ascorbic acid, tightly sealed and stirred for 30 min at RT. Then an olfactory test (smelling) was done and sensorically a moderate release of characteristically smelling isothiocyanates was determined. Dissolving only the single, not combined extracts a) and b) in drinking water did not result in any clearly recognizable smell characteristic for released isothyocyanates.

Example a) resulted in an extraction yield of 8.5 g (DER: 6:1), and a glucosinolate content in mg/g of 56.2, b) resulted in an extraction yield of 2.5 g (DER: 20:1), glucosinolate content not determined.

### Example c) - Preparation of glucosinolate - and myrosinase-comprising dry extracts from Armoracia rusticana

In a first step, 100 g dried and powdered horse radish root were extracted with 55% m/m ethanol in a ratio of 1:5 at RT under stirring at 300 rpm for 2h. Subsequently, the filtrate is filtered via a sheet filter and dried at 50°C and 200 to 1 mbar without auxiliary excipients in a rotary evaporator, resulting in partial extract c). In a second step, 20% m/m ethanol in a ratio of 1:5 is added to the remaining filter cake of the powdered horse radish root and extracted at RT under stirring at 300 rpm for 30 min. Subsequently, this mixture is adjusted under stirring with 1 molar NaOH to a pH of 8.0. Then the extracted plant particles are treated for 2 min with a Polytron^{®} at 5.000 RPM and stirred at RT and 300 rpm for further 30 min. The filtrate is filtered via a sheet filter and the filtrate is dried with 50 % m/m maltodextrin (relative to the dry extract content) in a rotary evaporator at 35-42°C and 200 to 1 mbar, resulting in partial extract d). Samples of partial extracts c) and d) are homogeneously mixed with addition of 0.5%m/m Aerosil^{®} and stored tightly sealed in an exsiccator. Partial extract c) resulted in an extraction yield of 21g (DER: 5:1) and partial extract d) resulted in an extraction yield of 5.2 g (DER: 19:1).

### Qualitative test for enzymatic activity of dry partial extracts c) and d)

1000 mg of the combined partial dry extracts were dissolved in 30 g drinking water comprising 2 mM ascorbic acid, tightly sealed and stirred for 30 min at RT. Then an olfactory test (smelling) was done and sensorically a very strong release of characteristically smelling isothiocyanates was determined. Dissolving only the single, not combined extracts c) and d) in drinking water did not result in any clearly recognizable smell characteristic for released isothyocyanates.

Example c) resulted in an extraction yield of 21 g (DEV: 5:1), and a glucosinolate content in mg/g of 81.2, and d) resulted in an extraction yield of 5.2 g (DEV: 19:1), glucosinolate content not determined.

### Examples e) and f) - Preparation of enriched glucosinolate- and myrosinase-comprising dry extracts from Armoracia rusticana

In a first step, 100 g dried and powdered horse radish root were extracted with 55% m/m ethanol in a ratio of 1:5 at RT under stirring at 300 rpm for 2h. Subsequently, the filtrate was filtered via a sheet filter and dried at 50°C and 200 to 30 mbar without auxiliary excipients in a rotary evaporator, to achieve a remaining volume of 40 to 50 ml. Then, 240 g 94% m/m ethanol are added and the extract is stirred for 30 min at RT and 300 rpm. The filtrate is filtered via a sheet filter and the filtrate is dried at 50°C and 200 to 1 mbar without auxiliary excipients in a rotary evaporator, resulting in partial extract e). The resulting extraction yield is 10.9 g (DER: 9:1), and a glucosinolate content in mg/g of 76.1.

In a second step, 5 parts 20%m/m ethanol are added to the remaining filter cake and extraction is repeated at RT with stirring and 300 rpm for 30 min. Then the mixture was adjusted under stirring with 1 molar NaOH to a pH of 8.5. The mixture is treated for 2min with a Polytron^{®} (PT 1200 E, Kinematica, Switzerland) at 5.000 RPM and stirring was continued at RT and 300 rpm for further 30min. The filtrate is filtered via sheet filter and the filtrate (476 g, extraction content: 2.73% m/m) is treated by molecular weight filtration with a 50 KD cut off cassette of the T-Serie (Pall^{®} Life Sciences, Centramate T-Series No OS050T02, Pall Corporation, USA) in counterflow. 380 g permeate were retrieved, the permeate side sealed and the retentate is obtained. The system is rinsed 4 times with portions of 50 g 20% m/m ethanol and retentate and the rinse fractions were combined. 280 g total retentate were gained with an extract content of 0.26% m/m. This corresponds to an extract yield of 0.73 g. 50% Maltodextrin (relative to dry substance content) was added to the retentate and it was dried in a rotary evaporator at 35-40°C and 200 to 1 mbar, resulting in partial extract f). Samples of partial extracts e) and f) were combined and homogeneously mixed with 0.1% m/m Aerosil^{®}, tightly sealed and stored in an exsiccator. Partial extract e) resulted in an extraction yield of 10.9 g, and partial extract f) resulted in an extraction yield of 1.46g.

### Qualitative test for enzymatic activity of dry partial extracts e) and f)

200 mg of the combined partial dry extracts were dissolved in 30 g drinking water comprising 2 mM ascorbic acid, tightly sealed and stirred for 30 min at RT. Then an olfactory test (smelling) was done and sensorically a very strong release of characteristically smelling isothiocyanates was determined.

Example e) resulted in an extraction yield of 10.9 g (DEV: 9:1), and a glucosinolate content in mg/g of 76.1, and f) resulted in an extraction yield of 1.46 g (DEV: 137:1), glucosinolate content not determined. Dissolving only the single, not combined extracts e) and f) in drinking water comprising 2 mM ascorbic acid, did not result in any clearly recognizable smell characteristic for released isothyocyanates.

## Claims

1. A dried plant extract mix comprising
(i) a dried extract component comprising mustard oil glycosides (glucosinolates), and
(ii) a dried extract component comprising active myrosinase enzymes, or isolated active myrosinase enzymes;
wherein the extract component (i) comprising mustard oil glycosides (glucosinolates) does not comprise active myrosinase enzymes.

2. The dried plant extract mix according to claim 1, wherein the dried plant extracts (i) and (ii) were prepared from different plant materials.

3. The dried plant extract mix according to claim 1, wherein the dried plant extracts (i) and (ii) were prepared from the same plant materials by sequential extraction, optionally a first extraction of the mustard oil glycosides, and a second extraction of active myrosinase enzymes from the retentate residue of the first extraction.

4. The dried plant extract mix according to any one of claims 1 to 3, wherein the dried plant extract comprises extract material from mustard oil (glucosinolate)- and myrosinase-comprising plants of the families Brassicaceae, Capparaceae, Caricaceae, Putranjivaceae and Tropaeolaceae.

5. The dried plant extract mix according to claim 4, wherein the dried plant extract mix comprises extract material from plants selected from the group consisting of
(vi) Brassicaceae species:
white cabbage, (Brassica oleracea var. capitata f. alba); brussels sprouts (Brassica oleracea var. gemmifera); broccoli (Brassica oleracea var. italica); cauliflower (Brassica oleracea var. botrytis); radish (Raphanus sativus); horseradish (Armoracia rusticana);
garden cress (Lepidium sativum); mustard (Sinapis alba, Sinapis nigra); rapeseed (Brassica napus); rucola (Arugula), Eruca sativa; Brassica oleracea var. gongylodes;
savoy cabbage (Brassica oleracea var. sabauda), kale (Brassica oleracea var. sabellica);
bok choy (Brassica rapa subsp. chinensis); wasabi (Eutrema japonicum); Daikon radish (raphanus sativus var. longipinnatus);
(vii) Capparaceae species: Capparis spinosa et var.;
(viii) Tropaeolaceae species: Tropaeolum majus et var.;
(ix) Caricaceae species : Carica papaya et var.; and
(x) Putranjivaceae : Drypetes separia et var.

6. The dried plant extract mix according to any one of claims 1 to 5, wherein the dried extract mix has
- a total content of mustard oil glycosides (glucosinolates) of 1 to 900, optionally 30 to 600, optionally 80 to 400 mg mustard oil glycosides per g dried extract component (i), and/or
- a total content of myrosinase enzymes of 0.01 units to 100.000 units, optionally 1 unit to 1000 unit, optionally 50 to 250 units per g dried extract component (ii).

7. The dried plant extract mix according to any one of claims 1 to 6, wherein the drug extract ratio (DER) of
- the extract component (i) of mustard oil glycosides (glucosinolates) is 2:1 to 50:1, optionally 5:1 to 16:1, optionally 4:1 to 10:1; and
- the extract component (ii) of myrosinase enzymes, or isolated myrosinase enzymes is 2:1 to 200:1, optionally 50:1 to 200:1, optionally 10:1 to 30:1.

8. A method for preparing the dried plant extract mix according to any one of claims 1 and 3 to 6 from the same plant material, comprising the following steps:
A Extraction of mustard oil glycosides (glucosinolate extract)
(a) extraction of plant material in 5 to 85, 10 to 67, 20 to 66, or 40 to 68 m/m aqueous organic solution, optionally alcohol, aldehyde or ketone-comprising aqueous organic solution, optionally comprising MeOH, EtOH, nPrOH, iPrOH, acetaldehyde, propanal, dimethylketone, ethylmethyl ketone, diethylketone, optionally EtOH, at 0 to 75 or 20 to 40°C, optionally at a pH of 3 to 10, or 6 to 9;
(b) separating the plant material from the liquid extract and evaporation of the liquid extract at 20 to 90 or 30 to 60°C, optionally at reduced pressure, optionally diluting with 0 to 35% m/m aqueous organic solution, optionally EtOH, filtration and evaporation of the filtrate;
(c) drying the filtrate, thereby producing extract component (i) (glucosinolates);
B Extraction of myrosinase enzymes (myrosinase extract)
(a) suspending the residue plant material from Extraction A in 0 to 68, 10 to 30, 15 to 25 or about 20 % m/m organic solution, optionally alcohol, aldehyde or ketone-comprising aqueous organic solution, optionally comprising MeOH, EtOH, nPrOH, iPrOH, acetaldehyde, propanal, dimethylketone, ethylmethyl ketone, diethylketone, optionally EtOH, optionally at a pH of 3 to 10, or at a pH of 7 to 9;
(b) optionally micronization of the suspended plant material;
(c) extraction of the suspended plant material at 0 to 50 or 10 to 42 C° for at least 5, 10, 20, or at least 30 min;
(d) filtration;
(e) optionally subjecting the filtrate of (d) to a 20 to 300, 40 to 100, 45 to 55, or an about 50 kD cut off membrane filtration;
(f) optionally adding a protein stabilizing agent to the filtrate of (d) or the retentate residue of (e), optionally maltodextrin;
(g) evaporation and drying the filtrate of (d), the retentate residue of (e) or (f), thereby producing extract (ii) (myrosinases);
C Combination of the dry glucosinolate and the dry myrosinase extract components (i) and
(ii), and optionally adding a dispersing agent, optionally a highly disperse silicon dioxide and optionally adding ascorbic acid or ascorbate, optionally further drying the plant extract mix.

9. A method for preparing the dried plant extract mix according to any one of claims 1, 2 and 4 to 6 from different plant materials, comprising the following steps:
A Extraction of mustard oil glycosides (glucosinolate extract)
(a) extraction of plant material in 0 to 95, 10 to 80, 30 to 75, or 40 to 70 m/m aqueous organic solution, optionally alcohol, aldehyde or ketone-comprising aqueous organic solution, optionally comprising MeOH, EtOH, nPrOH, iPrOH, acetaldehyde, propanal, dimethylketone, ethylmethyl ketone, diethylketone, optionally EtOH, at 5 to 80 or 20 to 60°C, optionally at a pH of 1 to 12, or 5 to 7;
(b) separating the plant material from the liquid extract and evaporation of the liquid extract at 20 to 90 or 30 to 60°C, optionally at reduced pressure, optionally diluting with 0 to 35% m/m aqueous organic solution, optionally EtOH, filtration and evaporation of the filtrate;
(c) drying the filtrate(s), thereby producing extract component (i) (glucosinolates);
B Extraction of myrosinase enzymes
(a) suspending plant material not used in Extraction A in 0 to 68, 10 to 30, 15 to 25 or about 20 % m/m aqueous organic solution, optionally alcohol, aldehyde or ketone-comprising aqueous organic solution, optionally comprising MeOH, EtOH, PrOH, iPrOH, acetaldehyde, propanal, dimethylketone, ethylmethyl ketone, diethylketone, optionally EtOH, optionally at a pH of 3 to 10, or at a pH of 5 to 9;
(b) optionally adding 1 to 20% m/m of a lipophilic phase relative to the aqueous phase of (a), optionally hydrocarbons, oils, waxes, optionally hexane, heptane, octane, plant oil, plant wax, bee wax;
(c) optionally micronization of the suspended plant material;
(d) extraction of the suspended plant material at 0 to 50 or 10 to 42 C° for at least 5, 10, 20 or at least 30 min;
(e) filtration, optionally microfiltration through a 0.01-10 µm cut off filter for separating the lipophilic phase of (b),
(f) optionally subjecting the filtrate of (e) to a 10 to 300, 40 to 100, 45 to 55, or an about 50 kD cut off membrane filtration and collecting the retentate comprising the myrosinases;
(g) optionally adding a protein stabilizing agent, optionally maltodextrin, to the filtrate of (e) or the retentate residue of (f);
(h) evaporation and drying the filtrate of (e), the retentate residue of (f) or (g), thereby producing extract component (ii) (myrosinases);
C Combination of the dry glucosinolate and the dry myrosinase extract components (i) and
(ii), and optionally adding a dispersing agent, optionally a highly disperse silicon dioxide, and /or optionally adding ascorbic acid or ascorbate, optionally further drying the plant extract mix.

10. A method for preparing the dried plant extract mix according to any one of claims 1 and 4 to 6 from (i) an extract comprising mustard oil glycosides (glucosinolates), and
(ii) isolated myrosinase enzymes, preparable from plant material, animal or microbial material (fungal, bacterial) or prepared recombinantly;
by combination of the extract (i) and the isolated myrosinase enzymes (ii), wherein extract (i) does not comprise active myrosinase enzymes.

11. The method according to claim 8, comprising the following steps
A Extraction of mustard oil glycosides (glucosinolate extract)
(a) extraction of Nasturtium plant material in 60 to 68% m/m EtOH, optionally at a pH of 2 to 4;
(b) filtration and evaporation of the filtrate of (a) to at least 20% of the extraction volume;
(c) re-dilution of filtrate (b) with 0 to 20% m/m EtOH, filtration, optionally filtration through a 1 to 50 kD cut off membrane;
d) evaporation and drying the filtrate of (c), thereby producing extract component (i) (glucosinolates);
B Extraction of myrosinase enzymes (myrosinase extract)
(a) resuspending the sediment from A in 0 to 68% m/m EtOH,
(b) optionally adding 1 to 20% m/m of a lipophilic phase relative to the aqueous phase;
(c) optionally micronization of the suspended plant material;
(d) extraction of the suspended plant material at 0 to 42C° for at least 5, 10, 20 or at least 30 min;
(e) optionally subjecting the filtrate of (d) to 20 to 300, 40 to 100, 45 to 55, or an about 50 kD cut off membrane filtration and collecting the retentate comprising the myrosinases;
(f) optionally adding a protein stabilizing agent, optionally maltodextrin, to the filtrate of (d) or the retentate residue of (e);
(g) evaporation and drying the filtrate of (d), the retentate residue of (e) or (f), thereby producing extract component (ii) (myrosinases);
C Combination of the dry glucosinolate and the dry myrosinase extract components (i) and
(ii), and optionally adding a dispersing agent, optionally a highly disperse silicon dioxide, and/or optionally ascorbic acid or ascorbate, optionally further drying the plant extract mix.

12. The method according to claim 8, comprising the following steps
A Extraction of mustard oil glycosides (glucosinolate extract)
(a) extraction of Armoracia plant material in 45 to 60% m/m EtOH, optionally at a pH of 2 to 10;
(b) filtration and evaporation of the filtrate of (a) to at least 20% of the extraction volume;
(c) re-dilution of filtrate (b) with 0 to 20% m/m EtOH, filtration, optionally filtration through a 1 to 50 kD cut off membrane;
d) evaporation and drying the filtrate of (c), thereby producing extract component (i) (glucosinolates);
B Extraction of myrosinase enzymes (myrosinase extract)
(a) Resuspending the sediment from A in 0 to 35%m/m EtOH, optionally at a pH of 5 to 9;
(b) optionally adding 1 to 20% m/m of a lipophilic phase relative to the aqueous phase;
(c) optionally micronization of the suspended plant material;
(d) extraction of the suspended plant material at 0-42C° for at least 5, 10, 20 or at least 60 min and filtration;
(e) optionally subjecting the filtrate of (d) to 20 to 300, 40 to 100, 45 to 55, or an about 50 kD cut off membrane filtration and collecting the retentate comprising the myrosinases;
(f) optionally adding a protein stabilizing agent, optionally maltodextrin, to the filtrate of (e) or the retentate residue of (f);
(g) evaporation and drying the filtrate of (d), the retentate residue of (e) or (f), thereby producing extract (ii) (myrosinases);
C Combination of the dry glucosinolate and the dry myrosinase extracts A and B, and
optionally adding a dispersing agent, optionally a highly disperse silicon dioxide and/or ascorbic acid, optionally further drying the plant extract mix.

13. The dried plant extract mix according to any one of claims 1 to 7, preparable by a method according to any one of claims 8 to 12.

14. A pharmaceutical composition comprising the dried plant extract mix according to any one of claims 1 to 7 and 13, and optionally at least one physiologically acceptable excipient, optionally selected from the group consisting of protein stabilizing agents, optionally selected from the group consisting of maltodextrin, trehalose, cyclodextrin, dextrose, dispersing agents, optionally selected from the group consisting of silicon dioxide, highly disperse silicon dioxide, calcium phosphate, calciumcarbonate, magnesiumcarbonate, citric acid, citrate, ascorbic acid, and ascorbate.

15. A dried plant extract mix according to any one of claims 1 to 7, 13 or a pharmaceutical composition according to claim 14 for use as a medicament, optionally for use in the treatment or prevention of a bacterial, viral or mycotic infection.
